# FASCICULE DE BREVET EUROPEEN

(11) **EP 0 346 247 B1**
(45) Date de publication et mention de la délivrance du brevet: **11.01.1995**
(21) Numéro de dépôt: 89420176.3
(22) Date de dépôt: 22.05.1989
(51) Int. Cl.: A61B 17/56

(54) **Clou centro-médullaire d'allongement progressif**
Intromedullärer Nagel zur progressiven Verlängerung
Progressively lengthening intramedullar nail

(30) Priorité: 09.06.1988 FR 8808099
(43) Date de publication de la demande: 13.12.1989
(73) Titulaire: MEDINOV SARL, F-42300 Roanne (FR)
(72) Inventeur: Grammont, Paul, F-21000 Dijon (FR); Guichet, Jean Marc, F-21003 Dijon cedex (FR)
(74) Mandataire: Dupuis, François

(56) Documents cités:
- EP-A- 0 212 192
- DE-A- 2 705 154
- GB-A- 2 137 884

## Description

L'invention se rattache au secteur technique des éléments de prothèses, utilisés notamment en chirurgie orthopédique.

On sait que, suite à des malformations ou anomalies congénitales, ou bien suite à des fractures, pertes de substance osseuse et autre, certains os, tels que l'humérus, le fémur, le tibia, ... sont trop courts, créant ainsi une asymétrie ou une hypométrie. Dans le cas des membres inférieurs, pour une asymétrie faible, inférieure à trois centimètres, on a recours à un traitement orthopédique, par adjonction au membre court par exemple d'une semelle, d'une chaussure, et autres appareillages orthopédiques. Pour une asymétrie ou une hypométrie plus importante, on dispose, pour obtenir un allongement du membre le plus court, de solutions chirurgicales, utilisant actuellement deux types de moyens :
- Les fixateurs externes : on opère en utilisant un appareillage externe dont certaines parties (par exemple broches métalliques, ...) traversent les tissus mous pour atteindre l'os auxquels elles sont solidarisées. L'appareillage apparaît à l'extérieur du membre. L'avantage d'un tel appareillage, qui est donc accessible de l'extérieur du membre, est de pouvoir agir sur ledit appareillage à des périodes déterminées pour provoquer, en conséquence, un certain degré d'allongement de l'os, d'une manière progressive, au niveau généralement de la diaphyse de l'os, après section de celle-ci. Cependant, des problèmes d'esthétisme, les difficultés pour l'utilisateur de supporter un tel appareillage, et les risques d'infection ne sont pas négligeables.
- Les fixateurs internes : la deuxième solution consiste à procéder d'une manière interne, c'est-à-dire qu'aucun élément n'apparaît à l'extérieur du membre. Pour cela, on utilise généralement des clous centro-médullaires ou des plaques d'ostéosynthèse, convenablement fixés à l'os. L'avantage de cette solution par rapport à la précédente, se trouve dans le fait qu'aucun élément n'apparaît à l'extérieur du membre. Par contre, il n'est pas possible de réaliser un allongement progressif de l'os. Un allongement assez important peut cependant être réalisé grâce à des interventions chirurgicales itératives consistant à chacune d'elles à obtenir un gain fixe, mais limité du fait de la limite élastique et plastique des tissus mous à l'étirement. En cas d'allongement important non progressif, le comblement de l'écart dimensionnel est aléatoire et nécessite des adjuvants à l'ostéosynthèse (greffes, ...) qui diminuent les propriétés mécaniques et physiologiques de l'os allongé.

Il n'existe actuellement aucun système de fixation interne strict (inclus entièrement dans l'organisme) qui permette d'obtenir un allongement de membre d'une manière progressive, avec un mécanisme incorporé au système de fixation mu soit automatiquement, soit par mobilisation externe du membre.

L'invention s'est fixée pour but de créer ce nouveau système d'allongement qui combine les avantages d'allongement progressif modulable à souhait des fixateurs externes, et les avantages des fixateurs internes, sans pour autant présenter les inconvénients de ces deux systèmes de fixation.

Le problème posé est donc de concevoir une solution selon laquelle les moyens utilisés et aptes à permettre un allongement de l'os à traiter, sont, d'une part, fixés d'une manière interne sans présenter une seule partie apparaissant à l'extérieur du membre et, d'autre part, conformés pour agir d'une manière progressive et modulable sur le degré d'allongement.

Pour résoudre ce problème, il a été conçu et mis au point un clou centro-médullaire interne à l'organisme agencé pour rendre solidaire au moins deux parties d'un même os et qui es remarquable en ce qu'il comprend, à l'intérieur de l'os concerné :
- un fourreau fixé dans l'une des parties d'os,
- à l'intérieur dudit fourreau étant monté avec capacité de déplacement coulissant en translation, un tube fixé dans l'autre partie de l'os,
- ledit tube étant accouplé à une douille filetée vissée dans un alésage taraudé du fourreau,
- ladite douille présentant à chaque extrémité, des agencements, coopérant, d'une part, avec une partie du tube et, d'autre part, avec des moyens de blocage,
- les moyens de blocage et lesdits agencements autorisent, lorsqu'une partie d'os est soumise à un couple rotatoire, dans un sens, l'entrainement en rotation de ladite douille pour déplacer concomitamment en translation le tube et, dans l'autre sens, le blocage en rotation de la douille pour interdire le déplacement en translation du tube.

Les agencements de la portée filetée sont constitués par des systèmes à cliquets disposés facialement à chacune des extrêmités de ladite portée, chaque système de cliquet étant inversé.

Les moyens de blocage, qui n'ont pas capacité de se déplacer en rotation, mais en translation par rapport au fourreau, et qui coopèrent avec la douille sont constitués par deux tiges cylindriques complémentaires représentant chacune une portée avec un méplat, lesdites portées étant imbriquées. Lesdites portées imbriquées constituent à la coupe un rond. Une desdites tiges permet le blocage rotatoire des moyens de blocage par rapport au fourreau, l'autre tige est agencée en combinaison avec la douille filetée.

La douille filetée, le tube coulissant et les moyens de blocages sont maintenus en pression par une vis, rivet ou autre moyen apte à remplir cette fonction de mise en pression.

La vis ou le moyen de mise en pression est engagée à l'intérieur du tube coulissant et de ladite douille pour être vissée ou fixée dans une des tiges cylindriques - -, la tête de vis ou un épaulement du moyen de mise en pression coopérant avec un organe élastique en appui sur un épaulement de l'alésage du tube.

Dans une autre forme de réalisation, les moyens de blocage qui coopèrent avec la douille sont constitués par un système à clavette coopérant avec un système à rainure formé dans l'alésage du fourreau.

Avantageusement, le système de blocage à clavette comprend un corps cylindrique portant au moins une clavette engagée dans une ou des rainures, ledit corps coopérant avec la douille en y étant maintenu en pression par l'organe sous forme d'un système axial de mise en contrainte du type vis, rivet ou autre engagélibrement dans l'épaisseur du corps et de ladite douille en étant fixé dans une partie du tube, ledit système coopérant avec un organe élastique.

Suivant une autre caractéristique, le tube et le fourreau présentent en combinaison des agencements complémentaires aptes à limiter le déplacement angulaire relatif dudit tube et dudit fourreau.

Le tube coulissant d'une part, et l'une des extrémités du fourreau, ainsi que l'extrémité de la tige cylindrique opposée à la douille, d'autre part, présentent les agencements pour la fixation du clou dans l'os.

Pour résoudre le problème posé de solliciter légèrement en compression le cal osseux, ce qui s'avère très profitable pour accélérer la densité de l'os en fin d'allongement sans pour autant perturber la phase d'allongement en tant que tel, l'un des organes de fixation est engagé angulairement dans l'épaisseur du fourreau dans une lumière oblongue au-travers d'une douille montée à libre coulissement dans ledit fourreau pour permettre, en combinaison avec un organe élastique, un déplacement linéaire limité en translation dudit fourreau par rapport à l'organe de fixation.

Pour résoudre un autre problème d'interdire tout mouvement d'allongement relatif en fin de traitement du clou, le fourreau et le tube présentent en combinaison des moyens conformés pour être commandés à partir de l'extérieur du membre, ou commandés automatiquement pour l'allongement voulu atteint avec commande incorporée audit clou, ou simple blocage mis en place au niveau du tube initialement positionné de façon à bloquer le glissement du tube coulissant dans le fourreau une fois l'allongement atteint, lesdits moyens étant aptes à assurer le blocage angulaire et longitudinal du tube coulissant et du fourreau.

L'invention est exposée ci-après plus en détail à l'aide des dessins annexés dans lesquels :

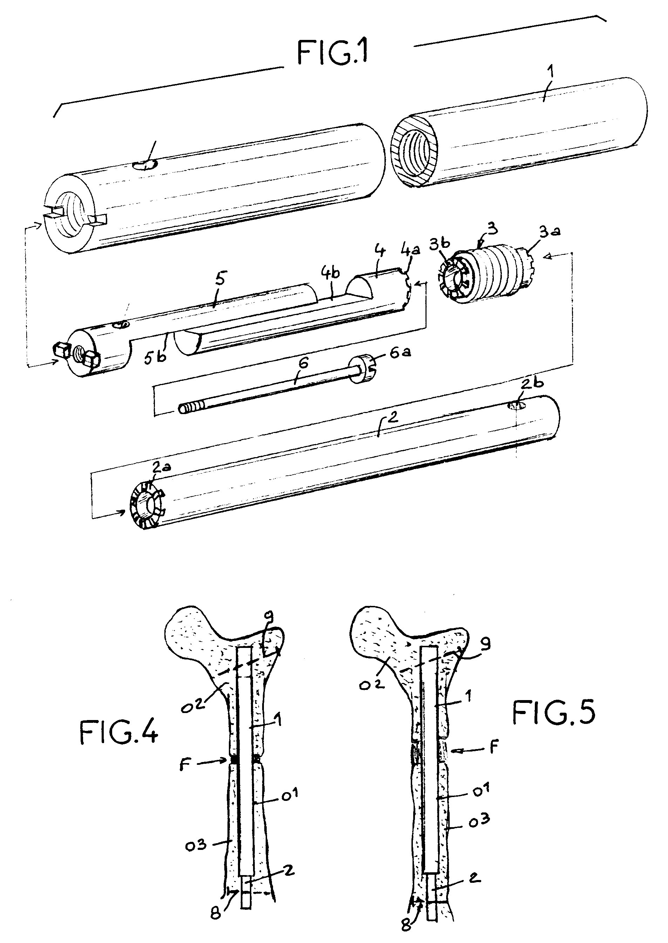

La figure 1 est une vue en perspective montrant les différents éléments constitutifs du clou selon l'invention.

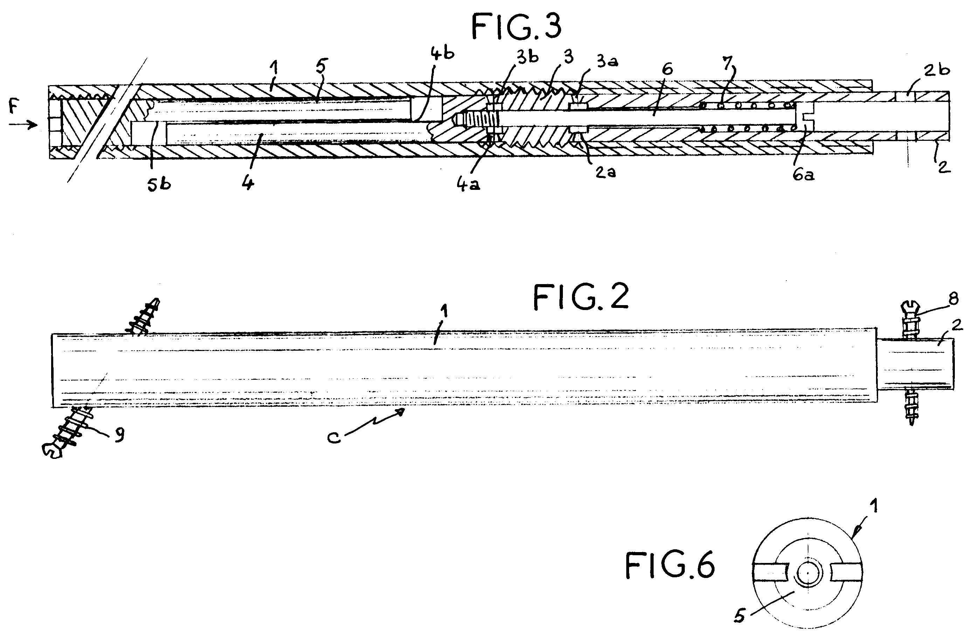

La figure 2 est une vue de face du clou.
La figure 3 est une vue en coupe longitudinale du clou.
Les figures 4 et 5 sont des vues à caractère schématique montrant la fixation et le principe de fonctionnement du clou.
La figure 6 est une vue en bout selon flèche F selon la figure 3.
La figure 7 est une vue en coupe longitudinale du clou selon une autre forme de réalisation.
La figure 8 est une vue en coupe transversale considérée selon la ligne 8-8 de la figure 7.
La figure 9 est une vue transversale selon la ligne 9-9 de la figure 7.

Afin de rendre plus concret l'objet de l'invention, on le décrit maintenant d'une manière non limitative en se référant aux figures des dessins.

Le clou désigné dans son ensemble par (C) est destiné à être positionné à l'intérieur du canal médullaire de l'os en vue de provoquer son allongement et de créer ainsi un écart dimensionnel entre les fragments osseux pour supprimer le raccourcissement dû par exemple à un problème congénital, une fracture, perte de substance osseuse.

Par exemple, le clou (C) sera fixé généralement au niveau de la diaphyse, de part et d'autre du fragment d'os coupé.

Le clou comprend un fourreau tubulaire (1) à l'intérieur duquel est monté avec capacité de déplacement en translation un tube (2). Selon l'invention, le fourreau (1) et le tube (2) sont assujettis à des moyens (3), (4) et (5) aptes à provoquer sous un effet de rotation par une manoeuvre externe de la partie distale du fragment coupé, dans un sens, le déplacement relatif en translation du fourreau et du tube correspondant à un allongement et, dans l'autre sens, le blocage dans la position précitée du tube (2) et du fourreau (1).

Dans la forme de réalisation illustrée, le tube (2) est asservi par une douille filetée (3) vissée dans l'alésage du fourreau (1) qui est taraudé en conséquence, en totalité ou en partie. Compte tenu du but recherché, cette douille (3) est agencée en combinaison d'une part avec le tube coulissant (2) et, d'autre part, avec les moyens de blocage (4 - 5) pour être entraînée en rotation dans un certain sens de rotation appliqué au membre, ce qui correspond à un déplacement du tube (2), et bloquée en rotation dans un sens inverse pour ne pas entraîner en translation ledit tube.

A cet égard, chacune des extrémités de la douille filetée (3) présente facialement des systèmes à cliquets (3a) et (3b). Chacun des systèmes (3a) et (3b) à une denture inverse pour créer des effets opposés comme indiqué ci-après.

L'un de systèmes à cliquet (3a) de la douille (3), coopère avec une denture complémentaire (2a) formée facialement en bout d'une des extrémités du tube (2). L'autre système à cliquet (3b) coopère avec une denture complémentaire (4a) formée facialement en bout d'un ensemble composé de deux tiges cylindriques complémentaires (4 et 5) constituant les moyens de blocage. Dans ce but, chacune des tiges (4) et (5) présente une portée semi-circulaire (4b) et (5b) lesdites portées étant aptes à être imbriquées pour autoriser en conséquence le coulissement relatif desdites tiges et leur blocage angulaire en rotation.

L'ensemble des éléments constitués par le tube (2), la douille (3) et la tige (4), sont maintenus en pression les uns par rapport aux autres, au moyen d'une vis (6). Cette vis (6) est engagée librement à l'intérieur du tube et de la douille (3), pour être vissée dans la tige cylindrique (4). La tête (6a) de la vis (6) est en appui sur un ressort de compression (7) monté dans l'alésage du tube (2) en appui sur un épaulement (2a).

L'extrémité débordante du tube (2), à l'opposé de la douille (3) présente transversalement un trou débouchant (2b) ou autre agencement pour le passage d' un organe de fixation du type vis (8). De la même façon, l'extrémité opposée du fourreau (1) et la tige (5) sont percées transversalement de part en part, pour le passage d'un organe de fixation du type vis (9).

Dans une autre forme de réalisation, le dispositif de blocage en rotation composé par les tiges (4 et 5) est remplacé par un système à clavette (10) coopérant avec un système à rainure (1a) formé dans l'alésage du fourreau (1) pour permettre le guidage en translation tout en verrrouillant la rotation.

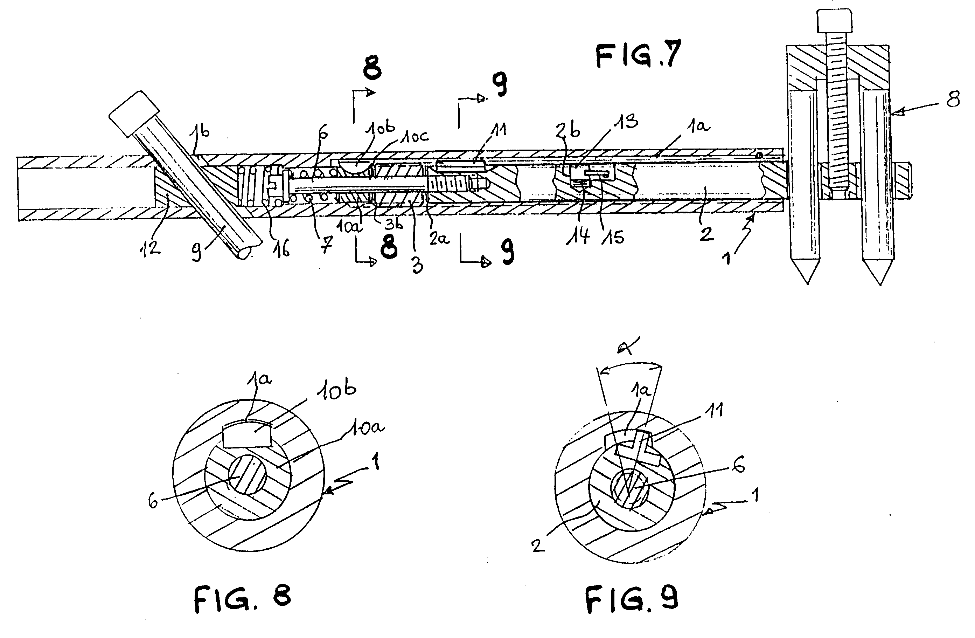

Comme le montrent notamment les figures 7 et 8, le système de blocage à clavette (10) comprend un corps cylindrique (10a) portant la clavette (10b) engagée dans la rainure (1a), ledit corps coopérant par l'une de ses faces (10c) avec la denture de l'un des systèmes à cliquet (3b) de la douille (3). Comme précédemment, l'autre système à cliquet (3a) de la douille (3) coopère avec la denture complémentaire (2a) du tube (2). De même, l'ensemble des éléments (10), (3) et (2) sont maintenus en pression par la vis (6) assujettie au ressort de compression (7). La vis (6) est engagée librement dans l'épaisseur du corps (10a) et de la douille (3) en étant vissée dans une partie du tube (2).

Avantageusement la rainure (1a) peut être utilisée pour limiter la rotation du tube (2) par rapport au fourreau (1) ou inversement. Dans ce but, le tube (2) présente une clavette (11) ou organe équivalent, apte à coopérer avec la rainure (1a) avec capacité de déplacement angulaire limite (α) sous l'effet d'entraînement dudit tube. Ce déplacement angulaire (α) est déterminé pour correspondre à la valeur d'une dent du système à cliquet de la douille (3) (figure 9).

Dans la forme de réalisation illustrée figure 7, l'organe de fixation (9) est engagé angulairement dans l'épaisseur du fourreau (1) dans une lumière oblongue (1b) et au-travers d'une douille (12) montée à libre coulissement à l'intérieur dudit fourreau. La douille (12) est assujettie à un ressort (16).

Il apparaît donc que le fourreau (1) est susceptible d'être déplacé linéairement en translation par rapport à l'organe de fixation (9) d'une valeur limitée. Dans ces conditions, à chaque pas, le cal osseux sera sollicité légèrement en compression, ce qui s'avère très profitable pour accélérer la densification de l'os en fin d'allongement, sans pour autant perturber la phase d'allongement en tant que telle.

Eu égard aux agencements du clou centro-médullaire aussi, il en résulte le fonctionnement suivant :
Le clou (C) est introduit d'une manière parfaitement connue, à l'intérieur du canal médullaire (01) de l'os à traiter (0) qui a été sectionné au niveau du foyer d'ostéotomie (F). On a repéré par (02) et (03) les deux parties d'os situés de part et d'autre du foyer d'ostéotomie.

Dans ces conditions, le clou est fixé de part et d'autre du foyer d'ostéotomie au moyen des vis (8) et (9) engagées respectivement dans les parties d'os (02) et (03) en coopérant avec l'extrémité débordante du tube (2) d'une part, le fourreau (1), d'autre part (figure 4). Il apparaît donc que sous un effet de rotation, par manoeuvre externe de la partie distale du fragment coupé, les parties (02) et (03) seront soumises à un couple rotatoire. Le fourreau (1) par exemple demeurant fixe en rotation, sous l'effet du couple, le tube (2) et concomitamment la douille filetée (3), seront entraînés en rotation, ce qui aura pour effet de provoquer le déplacement en translation dudit tube (2) correspondant à un allongement de la longueur du clou en fonction du pas du filetage de la douille.

L'entraînement concomitant en rotation du tube (2) et de la douille, dans un certain sens du couple de rotation appliqué, résulte du système d'encliquetage (2a) - (3a) qui est en position d'accouplement par son profil de denture, tandis que l'autre système d'encliquetage (3b) - (4a) est en position libre de non entraînement.

Inversement, dans l'autre sens du couple de rotation, le système d'encliquetage (2a) - (3a) se trouve en position libre de sorte que la rotation du tube (2) sera sans effet sur la douille (3), qui par ailleurs est bloquée en rotation par le système de tiges (4 - 5), ou le système de clavette (10) - rainure (1a), interdisant par conséquent le déplacement en translation inverse du précédent, dudit tube (2).

On voit donc qu'à chaque effet de mouvement rotatoire appliqué sur le membre, il y aura un déplacement en translation relatif du tube (2) et du fourreau (1), correspondant à un allongement du clou et par conséquent de l'os (figure 5).

Le but recherché est donc bien atteint étant donné que l'allongement s'opèrera d'une manière progressive et volontaire tout en respectant le problème initialement posé de n'avoir aucun élément externe.

A noter qu'en fin de traitement, lorsque la longueur désirée de l'os est atteinte, on prévoit d'inhiber la fonction d'allongement du clou tel que défini afin de lui conférer à demeure une position de blocage en translation. Par exemple, on peut envisager de pratiquer une intervention chirurgicale minime pour simplement poser une goupille ou autre moyen apte à bloquer en rotation le tube (2) par rapport au fourreau (1).

Par exemple, un doigt (13) assujetti à un ressort (14) est monté dans un logement (2b) du tube (2) en étant apte à coopérer avec la rainure (1a). Ce doigt est bloqué en position escamotée, c'est-à-dire en position de dégagement de la rainure (1a), par une goupille escamotable (15) actionnée par un aimant ou autre à partir de l'extérieur du membre. Lorsque la goupille est dégagée, sous l'effet de détente du ressort (14), le doigt (13) est engagé dans la rainure provoquant ainsi le blocage angulaire du tube (2) par rapport au fourreau (1) pour empêcher par conséquent tout mouvement d'allongement.

On peut bien évidemment envisager d'autres systèmes aptes à remplir la même fonction. Par exemple, le doigt peut être actionné par un mécanisme incorporé au clou et déclenché automatiquement, sans pour cela exclure tout système bloquant simplement les mouvements relatifs du tube coulissant dans le fourreau lorsque l'allongement voulu est atteint.

## Revendications

1. Clou centro-médullaire interne à l'organisme agencé pour rendre solidaire au moins deux parties (O2) et (O3) d'un même os (O), caractérisé en ce qu'il comprend, à l'intérieur de l'os concerné :
- un fourreau (1) fixé dans l'une des parties d'os (O2),
- à l'intérieur dudit fourreau étant monté avec capacité de déplacement coulissant en translation, un tube (2) fixé dans l'autre partie (O3) de l'os,
- ledit tube étant accouplé à une douille filetée (3) vissée dans un alésage taraudé du fourreau (1),
- ladite douille (3) présentant à chaque extrémité, des agencements (3a) (3b), coopérant, d'une part, avec une partie du tube (2) et, d'autre part, avec des moyens de blocage (4) (5) ou (10),
- les moyens de blocage (4) (5) ou (10) et lesdits agencements (3a) (3b) autorisent, lorsqu'une partie d'os (O2, O3) est soumise à un couple rotatoire, dans un sens, l'entrainement en rotation de ladite douille (3) pour déplacer concomitamment en translation le tube (2) et, dans l'autre sens, le blocage en rotation de la douille pour interdire le déplacement en translation du tube (2).

2. Clou selon la revendication 1, caractérisé en ce que les agencements de la douille filetée (3) sont constitués par des systèmes à cliquets (3a - 3b) disposés facialement à chacune des extrémités de ladite douille, chaque système de cliquet étant inversé.

3. Clou selon la revendication 1, caractérisé en ce que les moyens de blocage qui coopèrent avec la douille (3) sont constitués par deux tiges cylindriques complémentaires (4 et 5) présentant chacune une portée avec un méplat, lesdites portées étant imbriquées.

4. Clou selon la revendication 1, caractérisé en ce que les moyens de blocage qui coopèrent avec la douille (3) sont constitués par un système à clavette (10) coopérant avec un système à rainure (1a) formé dans l'alésage du fourreau.

5. Clou selon les revendications 1 et 4, caractérisé en ce que la douille filetée (3), le tube coulissant (2) et les moyens de blocage (4 - 5) ou (10) sont maintenus en pression par un organe (6).

6. Clou selon la revendication 5, caractérisé en ce que l'organe de mise en pression (6) est un système axial de mise en pression du type vis engagé à l'intérieur du tube (2) et de la douille vis-à-vis dudit organe, pour être fixé dans une partie des moyens de blocage (4 - 5), ledit système coopérant avec un organe élastique, en appui sur un agencement du tube (2).

7. Clou selon la revendication 5, caractérisé en ce que le système de blocage à clavette (10) comprend un corps cylindrique (10a) portant au moins une clavette (10b) engagée dans une ou des rainure (1a), ledit corps (10a) coopérant avec la douille (3) en y étant maintenu en pression par l'organe (6) engagé librement dans l'épaisseur du corps (10a) et de ladite douille, en étant fixé dans une partie du tube (2).

8. Clou selon la revendication 2, caractérisé en ce que l'extrémité du tube (2) présente facialement une denture complémentaire au système à cliquet (3a), de la douille conformée pour assurer l'entraînement concomitant dudit tube et de ladite douille.

9. Clou selon la revendication 2, caractérisé en ce que le système à cliquet (3b) coopère avec une denture complémentaire (4a), formée facialement en bout d'une partie de moyens de blocage (4 - 5) ou (10) en étant conformé pour permettre la libre rotation de la douille (3) et du tube (2).

10. Clou selon les revendications 1 et 4, caractérisé en ce que le tube (2) et le fourreau (1) présentent en combinaison des agencements complémentaires (11 et 1a) aptes à limiter le déplacement angulaire relatif dudit tube et dudit fourreau.

11. Clou selon la revendication 1, caractérisé en ce que le tube coulissant (2) d'une part, et l'une des extrémités du fourreau (1) d'autre part, présentent les agencements (2b, 8, 1b, 9) pour la fixation dudit clou à l'os.

12. Clou selon la revendication 11, caractérisé en ce que l'un des agencements de fixation (9) est engagé dans l'épaisseur du fourreau (1) dans une lumière oblongue (1b) au-travers d'une douille (12) montée à libre coulissement dans ledit fourreau pour permettre, en combinason avec un organe élastique (16) un déplacement linéaire limité en translation dudit fourreau par rapport à l'agencement de fixation.

13. Clou selon la revendication 1, caractérisé en ce que le fourreau (1) et le tube (2) présentent en combinaison des moyens (1a) et (13 - 15) conformés pour être commandés à partir de l'extérieur de l'organisme ou par un mécanisme ou moyen incorporé au clou, aptes à assurer le blocage angulaire du tube (2) et du fourreau pour interdire tout mouvement d'allongement relatif en fin de traitement.

## Claims

1. Intramedullary pin inside the organism devised to join at least two parts (O2) and (O3) of a single bone (O) characterised in that it comprises, inside the bone in question:
- a sleeve (1) fixed in one of the parts of the bone (O2),
- a tube (2) fixed in the other part (O3) of the bone being fitted inside said sleeve with the ability to make a sliding translational movement,
- said tube being coupled to a threaded bush (3) screwed into a tapped bore in the sleeve (1),
- each end of said bush (3) having features (3a) (3b) cooperating, on the one hand, with a part of tube (2) and, on the other hand, with the means of locking (4) (5) or (10),
- said means of locking (4) (5) or (10) and said features (3a) (3b) allowing, when one part of the bone (O2, O3) is subjected to rotational torque in one direction, rotational driving of said bush (3) in order to concomitantly produce translational movement of the tube (2) and, in the other direction, rotational locking of the bush in order to prevent translational movement of the tube (2).

2. Pin as claimed in claim 1 characterised in that the features of the threaded bush (3) consist of ratchet systems (3a-3b) arranged facially at each of the ends of said bush, each ratchet system being inverted.

3. Pin as claimed in claim 1 characterised in that the means of locking that cooperate with bush (3) consist of two complementary cylindrical rods (4 and 5) each having a bearing surface with a flat, said bearing surfaces being fitted together.

4. Pin as claimed in claim 1 characterised in that the means of locking that cooperate with bush (3) consist of a key system (10) cooperating with a slot system (1a) formed in the bore of the sleeve.

5. Pin as claimed in claims 1 and 4 characterised in that the threaded bush (3), the sliding tube (2) and the means of locking (4-5) or (10) are held in compression by a device (6).

6. Pin as claimed in claim 5 characterised in that the compression device (6) is an axial compression system of the type with a screw fitted inside the tube (2) and the bush vis-à-vis said device so that it can be fixed in part of means of locking (4-5), said system cooperating with an elastic device that rests against a feature of the tube (2).

7. Pin as claimed in claim 5 characterised in that the key locking system (10) comprises a cylindrical body (10a) carrying at least one key (10b) fitted in one or more slots (1a), said body (10a) cooperating with bush (3) by being secured there by compression by device (6) fitted freely in the thickness of the body (10a) and of said bush, being fixed in part of tube (2).

8. Pin as claimed in claim 2 characterised in that the end of tube (2) has toothing on its face that matches the ratchet system (3a) of the bush and is shaped to ensure concomitant driving of said tube and said bush.

9. Pin as claimed in claim 2 characterised in that the ratchet system (3b) cooperates with matching toothing (4a) formed facially at the end of one part of the means of locking (4-5) or (10) being shaped to allow free rotation of bush (3) and of tube (2).

10. Pin as claimed in claims 1 and 4 characterised in that the tube (2) and sleeve (1) have a combination of matching features (11 and 1a) suitable for limiting the relative angular displacement of said tube and of said sleeve.

11. Pin as claimed in claim 1 characterised in that the sliding tube (2) on the one hand and one of the ends of sleeve (1) on the other hand have features (2b, 8, 1b, 9) for fixing said pin to the bone.

12. Pin as claimed in claim 11 characterised in that one of the fixing features (9) is fitted in the thickness of sleeve (1) in an oblong slit (1b) through a bush (12) mounted so that it slides freely in said sleeve in order to allow, in combination with an elastic device (16), limited linear translational movement of said sleeve with respect to the fixing feature.

13. Pin as claimed in claim 1 characterised in that the sleeve (1) and tube (2) have a combination of means (1a) and (13-15) shaped to be controlled from outside the organism or by a mechanism or means built into the pin suitable to ensure angular locking of the tube (2) and of the sleeve in order to prevent any relative elongation movement at the end of treatment.

## Patentansprüche

1. Intrakorporaler Knochenmarksnagel, der dafür ausgebildet ist, mindestens zwei Teile (O2) und (O3) des gleichen Knochens (O) fest miteinander zu verbinden, dadurch gekennzeichnet, daß er innerhalb des betroffenen Knochens folgende Teile aufweist:
- eine Hülle (1), die in einem der Knochenteile (O2) befestigt ist,
- innerhalb dieser Hülle ein im anderen Teil (O3) des Knochens befestigtes Rohr (2), das in der Translationsbewegung gleitend verschiebbar ist,
- wobei das Rohr mit einer Gewindehülse (3) verbunden ist, die in einer Gewindebohrung der Hülle (1) verschraubt ist,
- wobei die Hülse (3) an jedem Ende Vorkehrungen (3a)(3b) besitzt, die zum einen mit einem Teil des Rohres (2) und zum anderen mit Sperrvorrichtungen (4) (5) oder (10) zusammenwirken;
- die Sperrvorrichtungen (4) (5) oder (10) und die Vorkehrungen (3a)(3b) bewirken, wenn ein Teil des Knochens (O2,O3) einem Drehmoment ausgesetzt ist, in einer Richtung die Drehmitnahme der Hülse (3) zur gleichzeitigen Translationsverschiebung des Rohres (2) und in der anderen Richtung die Drehsperrung der Hülse zur Verhinderung der Translationsverschiebung des Rohres (2).

2. Nagel nach Anspruch 1, dadurch gekennzeichnet, daß die Vorkehrungen der Gewindehülse (3) aus Klinksystemen (3a - 3b) bestehen, die vorne an jedem Ende der Hülse angeordnet sind, wobei jedes Klinksystem umgekehrt ist.

3. Nagel nach Anspruch 1, dadurch gekennzeichnet, daß die mit der Hülse (3) zusammenwirkenden Sperrvorrichtungen aus zwei komplementären zylindrischen Stangen (4 und 5) bestehen, die jeweils eine Auflagefläche mit einer Abflachung aufweisen, wobei die Auflageflächen aufeinanderliegen.

4. Nagel nach Anspruch 1, dadurch gekennzeichnet, daß die mit der Hülse (3) zusammenwirkenden Sperrvorrichtungen aus einem Keilsystem (10) bestehen, das mit einem in der Bohrung der Hülle gebildeten Nutsystem (1a) zusammenwirkt.

5. Nagel nach Anspruch 1 und 4, dadurch gekennzeichnet, daß die Gewindehülse (3), das Gleitrohr (2) und die Sperrvorrichtungen (4 - 5) oder (10) durch ein Bauteil (6) unter Druck gehalten werden.

6. Nagel nach Anspruch 5, dadurch gekennzeichnet, daß es sich bei dem druckausübenden Bauteil (6) um ein axiales Drucksetzungssystem handelt, bei dem eine Schraube in das Innere des Rohres (2) und der Hülse gegenüber dem Bauteil eingeführt wird, um in einem Teil der Sperrvorrichtungen (4 - 5) befestigt zu werden, wobei das besagte System mit einem elastischen Bauteil zusammenwirkt, das an einer Vorkehrung des Rohres (2) ansetzt.

7. Nagel nach Anspruch 5, dadurch gekennzeichnet, daß das Keilsperrsystem (10) einen zylindrischen Körper (10a) mit mindestens einem Keil (10b) besitzt, der in eine bzw. mehrere Nuten (1a) eingeführt ist, wobei der Körper (10a) mit der Hülse (3) zusammenwirkt, indem er durch das Bauteil (6) unter Druck gehalten wird, das frei beweglich in die Dicke des Körpers (10a) und der Hülse eingeführt und in einem Teil des Rohres (2) befestigt ist.

8. Nagel nach Anspruch 2, dadurch gekennzeichnet, daß das Endstück des Rohres (2) vorne ergänzend zum Klinksystem (3a) der Hülse eine Zahnung aufweist, die so ausgelegt ist, um für die gleichzeitige Mitnahme des Rohres und der Hülse zu sorgen.

9. Nagel nach Anspruch 2, dadurch gekennzeichnet, daß das Klinksystem (3b) mit einer komplementären Zahnung (4a) vorne am Ende eines Teils der Sperrvorrichtungen (4 - 5) oder (10) zusammenwirkt, indem es so ausgebildet ist, um die freie Drehung der Hülse (3) und des Rohres (2) zu ermöglichen.

10. Nagel nach Anspruch 1 und 4, dadurch gekennzeichnet, daß das Rohr (2) und die Hülle (1) eine Kombination der komplementären Vorkehrungen (11 und 1a) aufweisen, die geeignet sind, die relative Winkelverschiebung des Rohres und der Hülle zu begrenzen.

11. Nagel nach Anspruch 1, dadurch gekennzeichnet, daß einerseits das Gleitrohr (2) und andererseits eines der Enden der Hülle (1) die Vorkehrungen (2b, 8, 1b, 9) für die Befestigung des Nagels am Knochen aufweisen.

12. Nagel nach Anspruch 11, dadurch gekennzeichnet, daß eine der Befestigungsvorkehrungen (9) in der Dicke der Hülle (1) über eine frei gleitend in der Hülle angebrachte Hülse (12) in ein Schlitzloch (1b) eingeführt ist, um in Verbindung mit einem elastischen Bauteil (16) eine begrenzte lineare Translationsverschiebung der Hülle gegenüber der Befestigungsvorkehrung zu ermöglichen.

13. Nagel nach Anspruch 1, dadurch gekennzeichnet, daß die Hülle (1) und das Rohr (2) zusammen Vorrichtungen (1a) und (13 - 15) aufweisen, die so ausgebildet sind, um von außerhalb des Körpers oder durch einen in den Nagel eingebauten Mechanismus bzw. Vorrichtung betätigt zu werden, und die geeignet sind, für eine Winkelblockierung des Rohrs (2) und der Hülle zu sorgen, um bei Behandlungsende jegliche relative Dehnungsbewegung zu verhindern.
